# EUROPEAN PATENT SPECIFICATION

(11) **EP 3 145 556 B1**
(45) Date of publication and mention of the grant of the patent: **18.11.2020**
(21) Application number: 15725098.6
(22) Date of filing: 20.05.2015
(51) Int. Cl.: A61L 15/42, A61L 15/60, A61F 13/02, A61F 13/00

(54) **APERTURED HYDROGEL COMPOSITIONS AND WOUND DRESSINGS**
HYDROGELZUSAMMENSETZUNGEN MIT ÖFFNUNGEN UND WUNDAUFLAGE
COMPOSITIONS ET PANSEMENTS D'HYDROGEL À OUVERTURES

(30) Priority: 23.05.2014 GB 201409252
(43) Date of publication of application: 29.03.2017
(73) Proprietor: First Water Limited, Marlborough Wiltshire SN8 2RB (GB)
(72) Inventor: ANDREWS, Philip, Marlborough Wiltshire SN8 2RB (GB)
(74) Representative: Haseltine Lake Kempner LLP
(86) International application number: PCT/GB2015/051476
(87) International publication number: WO 2015/177540

(56) References cited:
- GB-A- 2 387 331
- US-A- 3 428 043
- DATABASE WPI Week 201381 Thomson Scientific, London, GB; AN 2013-W09509 XP002742348, -& CN 203 169 422 U (CHANGCHUN JIYUAN BIOTECH CO LTD) 4 September 2013 (2013-09-04)

## Description

### Field of the Invention

The present invention relates to compositions and wound dressings comprising hydrogels, which may be crosslinked, and their manufacture, and, in some embodiments, to composites, comprising hydrogels and one or more polymeric films and/or water-absorbent foam and/or aqueous solution swellable fibrous materials, having improved liquid permeability.

The expression "hydrogel" and like expressions, used herein, are not to be considered as limited to gels which contain only water, but extend generally to all hydrophilic gels and gel compositions, including those containing organic non-polymeric components in addition to water.

### Background of the Invention

Hydrogels are macromolecular networks swollen partially or to equilibrium with a suitable fluid, normally an aqueous fluid. Hydrogels can swell in water but do not dissolve. It is known that hydrogels are useful in a number of biomedical applications, including but not limited to wound and burns dressings, biomedical electrodes and skin adhesives, particularly because of their ability to donate and absorb fluid and hence maintain a moist but not wet environment.

It is known that the maintenance of a moist wound environment promotes the healing of wounds, especially burns and chronic wounds such as ulcers. However, it is also desirable to avoid excessive moisture or pooling of wound exudate on the wound, since liquid exudate causes maceration of skin adjacent to the wound and other difficulties. Furthermore, liquid exudate can leak from the wound site and contaminate clothes or bedding.

In practice, it is difficult to maintain the desired moisture level at the wound site because the rate of wound fluid production varies from wound to wound, and over time for any single wound. This can necessitate frequent dressing changes and a range of dressing types to treat different wounds.

EP-A-0123465 describes the use in surgical dressings of continuous polymer films formed from materials that have a higher moisture vapour permeability when the film is wet than when the film is dry.

EP-A-0875222 describes wound dressings comprising a non-swelling, water-impermeable apertured sheet having slits cut therein, wherein the apertured sheet is laminated to a water swellable foam layer. Absorption of wound fluid causes the foam layer to swell, and the resulting deformation opens the slits in the apertured sheet thereby increasing the liquid permeability of the apertured sheet.

EP-A-0122085 describes wound dressings having an apertured sheet of water swellable material laminated to a less water-swellable layer. Slits are cut in the apertured sheet. In use, differential swelling of the apertured sheet and the underlying layer causes the slits in the apertured sheet to open, thereby increasing the permeability of the apertured sheet to wound fluid.

WO2003011352 A1 describes a self-supporting apertured non reinforced sheet consisting essentially of a water-swellable hydrogel composition, wherein the area of the apertures is up to about 50 % of the area of the sheet before swelling and the area of the apertures increases on the absorption of fluid and decreases with subsequent loss of fluid.

The introduction of apertures into the pregelled self supporting hydrogels described in for example WO2003011352 A1 would be difficult to achieve using commercially viable die cutting processes due to the elasticty of such hydrogels. The presence of a reinforcing layer within or on the hydrogel is generally required to successfully die cut such hydrogels in a commerciably viable manufacturing process.

CN 203 169 422 describes a hydrogel wound dressing comprising a support material layer, a hydrogel layer and a release layer. The support material layer may be affixed to the surface of the hydrogel or incorporated within the hydrogel layer. There are penetrating holes in the hydrology which have a size of 5 to 30 mm.

Whilst the presence of a reinforcing layer, e.g. a scrim, within the hydrogel provides for a more structurally stable sheet, the present inventors have found that a decrease in aperture size occurs on swelling in aqueous fluids. This is quite different in behaviour to the aperture hydrogel sheet described in WO2003011352 A1, which did not contain a reinforcing member within the hydrogel, but in which the apertures increased in size on the hydrogel absorbing water. A reduction in aperture size may have been considered to be disadvantageous to the performance of apertured sheet hydrogel when used as a wound dressing. The present inventors have now found that an apertured sheet hydrogel comprising a reinforcing layer can have similar total fluid handling properties as a non apertured reinforced sheet hydrogel whilst providing for an improved liquid permeability, in particular viscous fluids, when the size of the apertures in the unswollen sheet are large enough, compared to the non apertured reinforced hydrogel sheet. A sheet hydrogel with sufficiently large apertures will facilitate a high evaporation rate of moisture. It is thought that the high evaporation rate contributes to minimising the swelling of the hydrogel around the apertures and consequently decreasing the extent of aperture closure. The advantage of the presence of a reinforcing layer is in the ease of conversion especially when taking a preformed reinforced hydrogel sheet and converting it, by die cutting, into an apertured material.

### Summary of the Invention

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

In a first aspect, the present invention provides a composition comprising:
a layer of water-swellable hydrogel, wherein the hydrogel has embedded therein a reinforcement sheet of liquid-water-permeable material,
wherein the layer of hydrogel has a wound-facing side and a non-wound-facing side and apertures extend through the layer of hydrogel from the wound-facing side to the non-wound-facing side and through the reinforcement sheet,
wherein the mean area of the apertures on at least one of the wound-facing side and non-wound-facing side of the hydrogel is at least 10 mm², and wherein a polymeric film is disposed over the non-wound-facing side of the layer of hydrogel, the film having an MVTR of at least 100 g/m²/24 hours, measured in accordance with BS EN 13726-2:2002.

In a second aspect, the present invention provides a method of forming a composition comprising:
providing a layer of water-swellable hydrogel, wherein the hydrogel has embedded therein a reinforcement sheet of liquid water-permeable material, the layer of hydrogel having a wound-facing side and a non-wound-facing side;
cutting apertures through the layer of water-swellable hydrogel and the reinforcement sheet embedded therein to form apertures that extend through the layer of hydrogel from the wound-facing side to the non-wound-facing side and through the reinforcement sheet,
wherein the mean area of the apertures on at least one of the wound-facing side and non-wound-facing side of the hydrogel is at least 10 mm², and wherein the method involves placing a polymeric film over the non-wound-facing side of the layer of hydrogel, the film having an MVTR of at least 100 g/m²/24 hours, measured in accordance with BS EN 13726-2:2002. The present invention further provides a composition formable by the method of the second aspect. The composition of the first aspect may be formable by the method of the second aspect.

### Brief Description of the Figures

Figure 1 shows schematically, from above either wound-facing or non-wound-facing sides, an embodiment of a layer of water swellable hydrogel having an array of apertures.
Figure 2 shows schematically a cross-sectional view of an embodiment of the composition or wound dressing described herein.

Further aspects, and preferable and optional features of the present disclosure are described below. Any preferable or optional feature may be combined with any other preferable or optional feature and/or with any aspect, unless otherwise stated.

Further described herein is a method of treating a wound in a human or non-human mammal, particularly a human, comprising contacting the wound for an effective period of time with a composition of the present invention, and wherein the wound-facing side of the layer of hydrogel is disposed closer to the wound than the non-wound facing side of the layer of hydrogel. In an embodiment, the wound-facing side of the hydrogel contacts the wound. In an alternative embodiment, one or more further layers, e.g. a further absorption layer as described herein, is/are disposed between the wound-facing side of the hydrogel and the wound.

Further described herein is the composition as described herein for use in a method of treating a wound in a human or non-human mammal, particularly a human, comprising contacting the wound for an effective period of time with a composition of the present invention, and wherein the wound-facing side of the layer of hydrogel is disposed closer to the wound than the non-wound facing side of the layer of hydrogel.

A wound to be treated using any of the aspects of the present invention may be of any type, e.g. acute or chronic. The wound may for example be a chronic ulcerous skin lesion, for example a malignant or pre-malignant chronic ulcerous skin lesion or a benign chronic ulcerous skin lesion.

The chronic ulcerous skin lesion may be selected from venous leg ulcers, venous foot ulcers, arterial leg ulcers, arterial foot ulcers, decubitus ulcers (e.g. pressure sores, bedsores), post-surgical ulcerous lesions and chronic burn lesions.

The chronic ulcerous skin lesion may be a high exudation lesion, a medium exudation lesion or a low exudation lesion.

Chronic skin lesions arise when a skin wound generally fails to follow an appropriate timely healing process to achieve the normal sustained and stable anatomic and functional integrity of the healed tissue. Generally speaking, a skin lesion which has failed to make at least substantial progress towards healing within a period of at least about three months, or which has become stable in a partially healed state for more than about three months, could be categorised as chronic, although even this general guide is not an absolute marker as the age and fitness of the patient, as well as other factors such as diseases or disorders suffered by the patient (for example, circulatory disorders), can significantly lengthen the normal healing process. A skin lesion which is unhealed after at least about one month, for example after at least about six months, can be categorised as chronic.

A chronic skin lesion is ulcerous where it involves focal loss of the epidermis and at least part of the dermis.

Malignant or pre-malignant chronic ulcerous skin lesions may arise in connection with a primary cancer of the skin, or with a metastasis to the skin from a local tumour or from a tumour in a distant site. They may be draining or non-draining. They may, for example, take the form of a cavity, an open area on the surface of the skin, skin nodules, or a nodular growth extending from the surface of the skin.

Benign chronic ulcerous skin lesions are not associated with cancer, and include venous leg ulcers, venous foot ulcers, arterial leg ulcers, arterial foot ulcers, decubitus ulcers (e.g. pressure sores, bedsores), post-surgical ulcerous lesions and chronic burn lesions. They may, for example, take the form of a cavity, an open area on the surface of the skin, skin nodules, or a nodular growth extending from the surface of the skin. Typically, they comprise an open granulating area on the surface of the skin.

Chronic ulcerous skin lesions are usually accompanied by other chronic symptoms apart from the failure of the normal healing process. Typical accompanying chronic symptoms include one or more of pain, exudation, malodour, excoriation, spreading of the wound, tissue necrosis, irritation and hyperkeratosis. Such symptoms can be extremely debilitating and embarrassing for patients, and can seriously harm the patient's quality of life. In severe cases, they can require amputation of limbs or even death. The composition according to the present invention may treat or relieve at least one of these symptoms.

Chronic ulcerous skin lesions can also be categorised according to their exudation. General categorisation is into the three categories "high exudation", "medium exudation" and "low exudation". Exudate management is a particularly difficult task for the caring professional attending to the patient. A balance needs to be struck between the desire to remove exudate to maintain the patient's quality of life at as high a level as possible, and maintenance of an appropriate level of fluid to prevent the lesion becoming too dry or too wet.

The method described herein may comprise the contacting of the wound with the composition of the present invention for an effective period of time to promote healing with simultaneous reduction in one or more of pain, exudation, malodour, excoriation, spreading of the wound, tissue necrosis, irritation and hyperkeratosis.

Further described herein is the use of the composition of the present invention in the preparation of a topical medicament for the treatment of a wound, for example a chronic skin lesion, in a human or non-human mammal, particularly a human.

The present invention further provides a wound dressing comprising, consisting essentially of or consisting of the composition of the present invention.

The mean area of the apertures on at least one of, optionally both of, the wound-facing side and non-wound-facing side of the hydrogel is at least 10 mm². "Mean area of the apertures" is calculated by dividing the total area occupied by the apertures on the wound-facing side or the non-wound-facing side of the layer of hydrogel by the number of apertures. In an embodiment, the mean area of the apertures on at least one of, optionally both of, the wound-facing side and non-wound-facing side of the layer of hydrogel is at least 20 mm². In an embodiment, the mean area of the apertures on at least one of, optionally both of, the wound-facing side and non-wound-facing side of the layer of hydrogel is from about 10 mm² to about 60 mm², optionally from about 20 mm² to about 50 mm², optionally from about 20 mm² to about 45mm², optionally from about 30 mm² to about 50 mm². For the avoidance of doubt, the size, and any other features of the apertures, are in the hydrogel that has capacity to absorb water; in an embodiment, the hydrogel having the apertures therein (and with the apertures being as defined herein) may contain at least some water, but is not at full water-absorption capacity. The water-absorption capacity of the hydrogel having the apertures therein will generally be at least about 30% by weight (i.e. the weight of water taken up and held at saturation will be at least about 30% of the weight of the hydrogel used), and may be as much as about 20000%. In an embodiment, the water-absorption capacity of the hydrogel having the apertures therein will at least about 100% by weight, optionally at least about 200% by weight, optionally at least about 300% by weight. In an embodiment, the water-absorption capacity of the hydrogel having the apertures therein will be between about 300% by weight and about 10000% by weight.

When the hydrogel of the hydrogel layer having apertures therein contains water, the water may be present in any suitable amount. The water content of the hydrogel of the hydrogel layer having apertures therein may be from about 0% by weight to about 95% by weight of the hydrogel, optionally from about 10% by weight to about 95% by weight of the hydrogel. The water content of the hydrogel of the hydrogel layer having apertures therein may be at least about 40% by weight, optionally at least about 50% by weight. The water content of the hydrogel of the hydrogel layer having apertures therein may be from about 10% by weight to about 40% by weight. The water content of the hydrogel of the hydrogel layer having apertures therein may be from about 50% by weight to about 95% by weight. The hydrogel of the hydrogel layer having apertures may contain water in the amounts stated above, the apertures are as defined herein, and the hydrogel has the capacity to absorb further water, for example in the amounts stated above; for example the hydrogel may have a water-absorption capacity of at least about 30% by weight, optionally at least about 100% by weight, optionally at least about 200% by weight, optionally at least about 300% by weight, optionally between about 300% by weight and about 10000% by weight.

The apertures may have any suitable shape, but preferably they are of a shape such that they remain open as the hydrogel swells on absorbing water. Preferably, they are of a shape such that the decrease in size of the apertures is limited when the hydrogel swells so as to maintain the total fluid handling capacity with increased liquid permeability compared to the non-apertured reinforced hydrogel. The apertures may have shapes, when viewed from the wound-facing side and/or non-wound-facing side of the layer of hydrogel, selected from round, oval, irregular or regular polygonal.

The cross-section of the apertures may be constant (tubular) through the thickness of the layer of hydrogel. In other embodiments, the apertures may taper through the thickness of the layer of hydrogel. This can result in apertures substantially in the form of truncated cones. The apertures will have an opening in the wound-facing side and the non-wound-facing side of the layer of hydrogel. At least some of the apertures, optionally all of the apertures, may have an opening in the wound-facing side of the layer of hydrogel that is larger, in area, than the opening in the non-wound-facing side of the layer of hydrogel. At least some of the apertures, optionally all of the apertures, may have an opening in the wound-facing side of the layer of hydrogel that is smaller, in area, than the opening in the non-wound-facing side of the layer of hydrogel.

Preferably, the total area of the apertures on the wound-facing side or non-wound-facing side of the layer of hydrogel is 60% or less of the total area of the wound-facing side or non-wound-facing side, respectively, of the layer of hydrogel. Preferably, the total area of the apertures on the wound-facing side or non-wound-facing side of the layer of hydrogel is about 10% to about 60%, optionally about 15 % to about 45% of the area of the layer of hydrogel before swelling, optionally about 20% to about 35% of the total area of the wound-facing side or non-wound-facing side, respectively, of the layer of hydrogel.

The apertured layer of hydrogel enables a moist wound environment to be maintained for prolonged periods, over a wide range of wound exudation rates. When the exudation rate is high, the apertured layer of hydrogel swells and the size of the apertures decreases but not to the point that the apertures close. The layer of hydrogel is thereby able to remove wound fluid to prevent excessive moisture in the wound without removal of the hydrogel or blocking of the apertures in the hydrogel. Furthermore, the hydrogel absorbs moisture vapour and functions as a humectant to preserve a moist wound contacting surface.

In certain embodiments, the area of the apertures on the wound-facing side or non-wound-facing side of the layer of hydrogel is decreased by at least about 10%, for example at least about 50% by swelling the layer of hydrogel with Solution A at 37°C for 24 hours. Typically, the layer of hydrogel has from about 1 to about 5 apertures per square 10 cm of the layer of hydrogel, for example from about 2 to about 5 apertures per 10 square cm. In certain embodiments the apertures are uniformly distributed over the surface of the layer of hydrogel, preferably in a regular pattern. In other embodiments the apertures are positioned in a central area of the layer of hydrogel with non-apertured hydrogel extending beyond the region where the apertures are located.

The apertured layer of hydrogel is reinforced. That is to say, the apertured layer of hydrogel has a embedded therein a reinforcement sheet of liquid-water-permeable material. The liquid-water-permeable material is typically a non-hydrogel material. The material is water permeable in that liquid water can pass from one side of the sheet to the other. The liquid-water-permeable material may be a perforated sheet, i.e. in the area between the apertures in the sheet of water-permeable material, there are perforations, the perforations typically each having an area less than 5 mm², preferably perforations each having an area less than 1 mm², preferably perforations each having an area less than 0.1 mm², optionally perforations each having an area less than 0.01 mm². The sheet of liquid-water-permeable material may be in the form selected from a film, a net, a woven fabric and a non-woven fabric. The reinforcement sheet of liquid-water-permeable material may be or comprise a scrim material, which may be in the form selected from a net, a woven fabric and a non-woven fabric, and optionally is formed from a non-water swellable material or optionally from a water swellable material or combination of both. Such a scrim material may be formed of a material that is natural in origin, synthetic in origin, or partly natural and partly synthetic. The reinforcement sheet, e.g. the scrim, may be formed from a material selected from polyolefins, polyamides, polyacrylates, and polyesters. The reinforcement sheet, e.g. the scrim, may be formed from a material selected from polyethylene and polypropylene. In an embodiment, the scrim material may comprise water swellable fibres for example calcium alginate fibres or carboxymethyl cellulose fibres or sodium polyacrylate fibres, such as those commercially available under the tradename Oasis™ from Acordis Technical Absorbents Limited. The scrim is preferably provided by introducing it into a laid down (e.g. cast) layer of a pre-gel liquid precursor for the hydrogel layer, before curing, so that the liquid pre-gel covers and surrounds the scrim. On curing of the liquid pre-gel, the hydrogel is thereby formed encapsulating the scrim material. In an embodiment, the scrim is preferably provided by introducing it into a laid down (e.g. cast) first layer of a pre-gel liquid precursor for the hydrogel layer, before curing, so that the liquid pre-gel covers and surrounds the scrim, curing the first layer of pre-gel and then laying down (e.g. casting) a second layer of a pre-gel liquid precursor for the hydrogel layer on the scrim and curing the second layer of pregel to form the layer of water-swellable hydrogel, having embedded therein the reinforcement sheet of liquid-water-permeable material in the form of a scrim. The scrim may have a weight of from 5 gsm to 50 gsm, optionally from 10 gsm to 40 gsm, preferably 10 gsm to 30 gsm, preferably from 15 to 25 gsm.

The layer of hydrogel having the apertures therein may comprise, consists essentially of or consist of the hydrogel and the reinforcement sheet of liquid-water-permeable material embedded in the hydrogel.

The composition or the layer of hydrogel according to the present invention may in some embodiments be adhesive on at least one side thereof, e.g. the wound-facing side thereof. In certain embodiments the hydrogel itself is adhesive. In other embodiments a medically acceptable pressure sensitive adhesive may be applied to a surface of the layer of composition, e.g. the wound-facing side thereof and/or any polymeric films associated with the layer of hydrogel.

According to the present invention, a polymeric film is disposed over the non-wound-facing side of the layer of hydrogel, and preferably the film has an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002. The polymeric film may have an MVTR of from 1000 g/m²/24 hours to 3000 g/m²/24 hours, measured in accordance with BS EN 13726-2:2002. In an embodiment, the polymeric film is continuous in that it extends over and covers each of the apertures in the layer of hydrogel; such a polymeric film may be termed a first film herein. In an embodiment, a further polymeric film, in addition to the first film, (which may also be termed a second film herein) is disposed over and in contact with the non-wound-facing side of the layer of hydrogel, the further polymeric film preferably having an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002, and the polymeric films are adhered together by a layer of adhesive. For the avoidance of doubt, where an MVTR of a film is given herein, the MVTR is of the film material absent of any apertures.

The polymeric film, e.g. the first and/or second polymeric film, may comprise or be a polyurethane film. Optionally, the polymeric film, e.g. the first and/or second polymeric film, which may be or comprise a polyurethane film, is breathable. The polymeric film, e.g. the first and/or second polymeric film, may have an MVTR of at least 10 g/m²/24 hours, optionally at least at least 50 g/m²/24 hours, optionally at least 100 g/m²/24 hours, optionally at least 200 gm²/24 hours, optionally at least 300 g/m²/24 hours, optionally at least 500 g/m²/24 hours, optionally at least 1000 g/m²/24 hours, with MVTR as measured by the method described in on BS EN 13726-2:2002. The polymeric film, e.g. the first and/or second polymeric film, may have an MVTR of from 100 g/m²/24 hours to 10000 g/m²/24 hours, optionally an MVTR of from 100 g/m²/24 hours to 4000 g/m²/24 hours.

In an embodiment, the composition or dressing further comprises a backing layer over the non-wound facing side of the layer of hydrogel. The first film described herein may be or form part of the backing layer. The backing layer preferably provides a barrier to passage of microorganisms through the dressing and further preferably blocks the escape of wound fluid from the dressing. The backing layer also provides for a reduction in the evaporation of moisture compared to when no backing layer is present. This reduction of the evaporation of moisture when combined with the water content of the hydrogel provides for the maintenance of a moist environment over the wound and overcomes the proposed problems of hydrogel sheets with large diameter apertures.

In an embodiment, the mean area of the apertures on at least one of the wound-facing side and non-wound-facing side of the layer of hydrogel is at least 10 mm²,
the layer of hydrogel has from about 1 to about 5 apertures per square 10 cm of the wound-facing side and/or non-wound-facing side,
the hydrogel is an ionic hydrogel, and
a polymeric film is disposed over the non-wound-facing side of the layer of hydrogel, the film having an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002, and optionally the polymeric film is continuous in that it extends over and covers each of the apertures in the layer of hydrogel.

Optionally, an absorbent layer is disposed between the layer of hydrogel and the polymeric film. The absorbent layer may comprise a foamed hydrophilic material. The foamed hydrophilic material may be selected from a foamed polyurethane and a foamed hydrogel.

Optionally, the layer of hydrogel is 3 mm or less in thickness, measured from the wound-facing side to the non-wound-facing side. Optionally, the layer of hydrogel is 2 mm or less, optionally 1 mm or less, in thickness, measured from the wound-facing side to the non-wound-facing side.

The present invention further provides the method of forming a composition as described above. The method may involve die cutting the apertures in the layer of hydrogel and, if present, a polymeric film layer thereon. In an embodiment, the method further involves placing a continuous polymeric film over the non-wound-facing side of the layer of hydrogel, optionally the film having an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002, wherein the polymeric film is continuous in that it extends over and covers each of the apertures in the layer of hydrogel.

Optionally, the method involves placing a polymeric film over the non-wound-facing side of the layer of hydrogel, the film optionally having an MVTR of at least 100 g/m²/24 hours, optionally at least 200 g/m²/24 hours, optionally at least 300 g/m²/24 hours, optionally at least 500 g/m²/24 hours, at least 600 g/m²/24 hours, measured in accordance with BS EN 13726-2:2002, wherein either the polymeric film is placed over the non-wound-facing side of the layer of hydrogel before the cutting of the apertures in the layer of hydrogel and apertures are cut in both the polymeric film and the layer of hydrogel or the polymeric film is placed over the non-wound-facing side of the layer of hydrogel after the cutting of the apertures in the layer of hydrogel and the polymeric film has apertures therethrough, at least some of which overlie the apertures of the layer of hydrogel; this method may further involve adhering a continuous polymeric film to the apertured polymeric film, and optionally the continuous polymeric film has an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002, wherein the polymeric film is continuous in that it extends over and covers each of the apertures in the layer of hydrogel and/or the apertured polymeric film. Optionally, the continuous polymeric film is adhered to the apertured polymeric film by means of a layer of adhesive, the layer of adhesive has apertures therein, at least some of which overlie the apertures in the layer of hydrogel and/or the apertures of the apertured polymeric film. In an embodiment, the continuous polymeric film is adhered to the apertured polymeric film by means of a layer of adhesive, the layer of adhesive being present on the polymeric film that is placed over the non-wound-facing side of the layer of hydrogel at the time at which holes are cut in the polymeric film, the holes being cut in both the polymeric film and the adhesive, and optionally the apertures in the layer of adhesive are approximately the same size, shape and in the same arrangement as the apertures in the layer of hydrogel and the apertures in the polymeric film, such that each aperture in the layer of adhesive has a corresponding aperture in the hydrogel and/or the polymeric film.

In an embodiment, the method further involves placing an absorbent layer and a polymeric film over the non-wound-facing side of the layer of hydrogel, optionally the film having an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002, the absorbent layer being disposed closer to the non-wound-facing side of the layer of hydrogel than the polymeric film. The absorbent layer may be the further absorbent layer as described herein.

The present invention further provides a wound dressing comprising an apertured reinforced layer of hydrogel according to the present invention.

In use, an adhesive layer may be disposed on at least a part of a wound-facing side of the backing layer, e.g. the first film described above, and the adhesive can act to adhere to the apertured hydrogel, and, if present any supporting layer, e.g. the second film described above, on the apertured hydrogel to the backing layer and/or can adhere the wound dressing to the skin of a user of the dressing.

Optionally an adhesive layer may be disposed on at least a part of the wound-facing side of the backing layer, which may be or comprise the first film as described above, and the adhesive can act to adhere to the apertured hydrogel, and, if present any supporting layer, e.g. the second film as described above, on the apertured hydrogel, to the backing layer and/or can adhere the wound dressing to the skin of a user of the dressing.

In certain embodiments the support layer comprises an apertured polymeric film, e.g. the second film as described above, with an apertured adhesive layer disposed on the non wound facing side of the polymeric film. The apertures in the polymeric film support layer and adhesive layer prefeably correspond to the size and location of the apertures in the layer of hydrogel.

Optionally, the dressing further comprises a layer of absorbent material (which may be termed an absorbent layer herein) disposed on a side of the apertured layer of hydrogel facing away from the backing layer; and preferably the absorbent layer does not comprise a hydrogel or a layer of hydrogel, apertured or non-apertured. Optionally, the absorbent layer comprises a water-absorbent open celled foam. Optionally, the absorbent layer comprises a water-absorbent, aromatic or aliphatic polyurethane open celled foam. An aromatic polyurethane is a polyurethane formed from monomers comprising an aromatic group. An aliphatic polyurethane is a polyurethane formed from monomers comprising an aliphatic group. In an embodiment, the absorbent layer extends across the gaps between the apertures of hydrogel.

Optionally, the dressing further comprises a layer of absorbent material (which may be termed an absorbent layer herein) disposed on a side of the apertured layer of hydrogel facing away from the wound; and preferably the absorbent layer does not comprise a hydrogel or a layer of hydrogel, apertured or non-apertured. Optionally, the absorbent layer comprises a water-absorbent open celled foam. Optionally, the absorbent layer comprises a water-absorbent, aromatic or aliphatic polyurethane open celled foam. An aromatic polyurethane is a polyurethane formed from monomers comprising an aromatic group. An aliphatic polyurethane is a polyurethane formed from monomers comprising an aliphatic group. In an embodiment, the absorbent layer extends across the gaps between the apertures of hydrogel.

Optionally, the absorbent layer comprises fibres. Optionally, the fibres form a gel when hydrated, which are sometimes termed gel-forming fibres in the art. Optionally, the fibres comprise an alginate, chitosan or a cellulose derivative or any combination thereof. Preferred types of fibre comprise calcium alginate (available from, for example, Foshan United Medical Technologies Ltd, China), and/or sodium polymethacrylate (available, for example, under the tradename Oasis™ from Technical Absorbents Limited). Optionally, the fibres comprise an alginate, optionally calcium alginate, containing in its polysaccharide chain guluronic and/or mannuronic acid, and optionally with a molar ratio of guluronic to mannuronic acid from 90:10 to 10:90, more preferably from 80:20 to 20:80 and even more preferably from 80:20 to 30:70. A particularly preferred fibre comprises an alginate comprising one or more of the following ions, calcium, sodium, zinc and silver. The cellulose derivative may comprise, for example, hydroxypropylmethyl cellulose or carboxymethyl cellulose.

### Hydrogel

The hydrogel in the dressing or composition preferably consists essentially of a cross-linked hydrophilic polymer of a hydrophilic monomer and optionally one or more comonomers, together with water and/or one or more organic plasticiser, and optionally together with one or more additives selected from surfactants, polymers, pH regulators, electrolytes, chloride sources, bioactive compounds and mixtures thereof, with less than about 10% by weight of other additives. Preferably the hydrogel is made from a precursor solution. The hydrogel of the layer of hydrogel typically has a continuous internal structure, in that, in the areas between the apertures described herein, it does not contain pores or have an internal cellular structure.

Preferably, the precursor solution is aqueous. The precursor solution may comprise aqueous solutions of one or more monomers that are ionic, non-ionic, amphoteric, zwitterionic or combinations thereof.

The precursor solution preferably contains one or more monomers capable on polymerisation of forming a three-dimensional matrix of cross-linked polymer molecules.

The expressions "polymer", "polymerisation" and like expressions, used herein, includes within its scope homopolymerisation and copolymerisation and the products thereof.

Optionally, the hydrogel is an ionic hydrogel, i.e. a hydrogel formed from ionic monomers. Optionally, the hydrogel, which may be an ionic hydrogel, comprises a hydrophilic polymer having multiple pendant sulphonyl groups, optionally with multiple pendant carboxylic groups. Optionally, at least some of the pendant sulphonyl groups are in salt form, for example associated with one or more cations, for example selected from sodium and potassium.

Examples of suitable monomers for use in forming the hydrogel, include: 2-acrylamido-2-methylpropane sulphonic acid or a substituted derivative thereof or a salt thereof (e.g. an ammonium or alkali metal salt such as sodium, potassium or lithium salts); acrylic acid or a substituted derivative thereof or a salt thereof (e.g. an alkali metal salt such as sodium, potassium or lithium salt); a polyalkylene glycol acrylate or a substituted derivative thereof; a polyalkylene glycol methacrylate or a substituted derivative thereof; acrylic acid (3-sulphopropyl) ester or a substituted derivative thereof or a salt thereof (e.g. an alkali metal salt such as sodium, potassium or lithium salt); diacetone acrylamide (N-1,1-dimethyl-3-oxobutyl-acrylamide); a vinyl lactam (e.g. N-vinyl pyrrolidone or a substituted derivative thereof); an optionally substituted N-alkylated acrylamide such as hydroxyethyl acrylamide; and an optionally substituted N,N-dialkylated acrylamide; and/or N-acryloyl morpholine or a substituted derivative thereof. Preferably, the polymerisable monomer is selected from the 2-acrylamido-2-methylpropane sulphonic acid, which is optionally in salt form, for example associated with one or more cations, for example selected from sodium and potassium; acrylic acid (3-sulphopropyl) ester, which is optionally in salt form, for example associated with one or more cations, for example selected from sodium and potassium; N-acryloyl morpholine; and hydroxyethyl acrylamide.

The hydrogel used in the present invention preferably comprises a plasticised three-dimensional matrix of cross-linked polymer molecules, and has sufficient structural integrity to be self-supporting even at very high levels of internal water content, with sufficient flexibility to conform to the surface contours of mammalian skin or other surface with which it is in contact.

The hydrogel generally comprises, in addition to the cross-linked polymeric network, an aqueous or non-aqueous plasticising medium including an organic plasticiser. This plasticising medium is preferably present in the same precursor solution as the monomer(s), although if desired it may be applied to the fibrous material separately from the monomer(s) but before polymerisation.

In the material to be polymerised, the precursor solution preferably comprises the monomer(s), cross-linking agent, plasticiser, which may be an organic plasticiser, and optionally water and other ingredients as desired. The polymerisation reaction is preferably a free-radical polymerisation with cross-linking, which may for example be induced by light, heat, radiation (e.g. ionising radiation), or redox catalysts, as is well known.

For example, the free radical polymerisation may be initiated in known manner by light (photoinitiation), particularly ultraviolet light (UV photoinitiation); heat (thermal initiation); electron beam (e-beam initiation); ionising radiation, particularly gamma radiation (gamma initiation); non-ionising radiation, particularly microwave radiation (microwave initiation); or any combination thereof. The precursor solution may include appropriate substances (initiators), at appropriate levels, e.g. up to about 5% by weight, more particularly between about 0.002% and about 2% by weight, which serve to assist the polymerisation and its initiation, in generally known manner.

Preferred photoinitiators include any of the following either alone or in combination:
Type I-α-hydroxy-ketones and benzilidimethyl-ketals e.g. Irgacure 651. These are believed on irradiation to form benzoyl radicals that initiate polymerisation. Photoinitiators of this type that are preferred are those that do not carry substituents in the *para* position of the aromatic ring. Examples include Irgacure184 and Daracur 1173 as marketed by Ciba Chemicals, as well as combinations thereof.

A particularly preferred photoinitiator is 1-hydroxycyclohexyl phenyl ketone; for example, as marketed under the trade name Irgacure 184 by Ciba Speciality Chemicals. Also preferred are (available under the trade name Daracur 1173) and mixtures of 1-hydroxycyclohexyl phenyl ketone and 2-hydroxy-2-propyl phenyl keton, for example mixtures of Irgacure 184 and Daracur 1173.

Photo-polymerisation is particularly suitable, and may be achieved using light, optionally together with other initiators, such as heat and/or ionizing radiation. Photoinitiation will usually be applied by subjecting the pre-gel reaction mixture containing an appropriate photoinitiation agent to ultraviolet (UV) light. The incident UV intensity, at a wavelength in the range from 240 to 420nm, is typically greater than about 10mW/cm². The processing will generally be carried out in a controlled manner involving a precise predetermined sequence of mixing and thermal treatment or history.

The UV irradiation time scale should ideally be less than 60 seconds, and preferably less than 10 seconds to form a gel with better than 95% conversion of the monomers. Those skilled in the art will appreciate that the extent of irradiation will be dependent on a number of factors, including the UV intensity, the type of UV source used, the photoinitiator quantum yield, the amount of monomer(s) present, the nature of the monomer(s) present and the presence of polymerisation inhibitor.

After completion of the polymerisation and conversion into a wound dressing, the hydrogel is preferably sterilised in conventional manner.

If desired, certain ingredients of the hydrogel may be added after the polymerisation and optional cross-linking reaction. However, it is generally preferred that substantially all of the final ingredients of the hydrogel are present in the precursor solution, and that - apart from minor conventional conditioning or, in some cases, subsequent modifications caused by the sterilisation procedure - substantially no chemical modification of the hydrogel takes place after completion of the polymerisation reaction.

### Monomers

Optional substituents of the monomers used to prepare the hydrogels used in the present invention may preferably be selected from substituents which are known in the art or are reasonably expected to provide polymerisable monomers which form hydrogel polymers having the properties necessary for the present invention. Suitable substitutents include, for example, lower (e.g. C₁ to C₅) alkyl, hydroxy, halo and amino groups.

Particularly preferred monomers include: the sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A); acrylic acid (3-sulphopropyl) ester potassium salt, commonly known as SPA or SPAK (SPA or SPAK is available commercially in the form of a pure solid from Raschig); N-acryloyl morpholine; and hydroxyethyl acrylamide.

### Cross-linking Agents

Conventional cross-linking agents can be used to provide the necessary mechanical stability and to control the adhesive properties of the hydrogel. The amount of cross-linking agent required will be readily apparent to those skilled in the art such as from about 0.01% to about 0.5%, particularly from about 0.05% to about 0.4%, most particularly from about 0.08% to about 0.3%, by weight of the total polymerisation reaction mixture. Typical cross-linkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide.

### Organic Plasticisers

One or more organic plasticisers may be present in the hydrogel and/or the hydrogel precursor solution. The one or more organic plasticisers, when present, may suitably comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is the preferred plasticiser. An alternative preferred plasticiser is the ester derived from boric acid and glycerol. When present, the organic plasticiser may comprise up to about 45% by weight of the hydrogel composition.

### Surfactants

Any compatible surfactant may optionally be used as an additional ingredient of the hydrogel composition. Surfactants can lower the surface tension of the mixture before polymerisation and thus aid processing. The surfactant or surfactants may be non-ionic, anionic, zwitterionic or cationic, alone or in any mixture or combination. The surfactant may itself be reactive, i.e. capable of participating in the hydrogel-forming reaction. The total amount of surfactant, if present, is suitably up to about 10% by weight of the hydrogel composition, preferably from about 0.05% to about 4% by weight.

In a preferred embodiment of the invention the surfactant comprises at least one propylene oxide/ethylene oxide block copolymer, for example such as that supplied by BASF Pic under the trade name Pluronic P65 or L64.

### Other additives

The hydrogel may include one or more additional ingredients, which may be added to the pre-polymerisation mixture or the polymerised product, at the choice of the skilled worker. Such additional ingredients may be selected from additives, including, for example, water, organic plasticisers, surfactants, polymeric material (hydrophobic or hydrophilic in nature, including proteins, enzymes, naturally occuring polymers and gums), synthetic polymers with and without pendant carboxylic acids, electrolytes, pH regulators, colorants, chloride sources, bioactive compounds and mixtures thereof. The polymers can be natural polymers (e.g. xanthan gum), synthetic polymers (e.g. polyoxypropylene-polyoxyethylene block copolymer or poly-(methyl vinyl ether *alt* maleic anhydride)), or any combination thereof. "Bioactive compounds" may indicate any compound or mixture included within the hydrogel for some effect it has on living systems, whether the living system be bacteria or other microorganisms or higher animals such as the patient. Bioactive compounds that may be mentioned include, for example, pharmaceutically active compounds, antimicrobial agents, antiseptic agents, antibiotics and any combination thereof. Antimicrobial agents may, for example, include: souces of oxygen and/or iodine (e.g. hydrogen peroxide or a source thereof and/or an iodide salt such as potassium iodide) (see, for example Bioxzyme™ technology, for example in The Sunday Telegraph (UK) 26 January 2003 or the discussion of the Oxyzyme™ system at www.wounds-uk.com/posterabstracts2003.pdf); honey (e.g. active Manuka honey); antimicrobial metals, metal ions and salts, such as, for example, silver-containg antimicrobial agents (e.g. colloidal silver, silver oxide, silver nitrate, silver thiosulphate, silver sulphadiazine, or any combination thereof); or any combination thereof.

Hydrogels incorporating antimicrobial agents may, for example, be active against such organisms as *Staphylococcus aureus* and *Pseudomonas aeruginosa.*

Agents for stimulating the healing of wounds and/or for restricting or preventing scarring may be incorporated into the hydrogel. Examples of such agents include growth factors e.g. from GroPep Ltd, Australia or Procyte, USA (see, e.g. WO-A-96/02270; cell nutrients (see, e.g., WO-A-93/04691, the contents of which are incorporated herein by reference); glucose (see, e.g., WO-A-93/10795); an anabolic hormone or hormone mixture such as insulin, triiodothyronine, thyroxine or any combination thereof (see, e.g., WO-A-93/04691); or any combination thereof. Additional polymer(s), typically rheology modifying polymer(s), may be incorporated into the polymerisation reaction mixture at levels typically up to about 10% by weight of total polymerisation reaction mixture, e.g. from about 0.2% to about 10% by weight. Such polymer(s) may include polyacrylamide, poly-NaAMPS, polyethylene glycol (PEG), polyvinylpyrrolidone (PVP) or carboxymethyl cellulose.

The hydrogel in the composite of the present invention preferably consists essentially of a cross-linked hydrophilic polymer of a hydrophilic monomer and optionally one or more comonomer, together with water and/or one or more organic plasticiser, and optionally together with one or more additives selected from surfactants, polymers, pH regulators, electrolytes, chloride sources, bioactive compounds and mixtures thereof, with less than about 10% by weight of other additives.

The hydrogel may be formed by disposing a layer of precursor solution on a substrate, then curing the precursor solution to form the hydrogel, and cutting portions of the hydrogel and removing them to form the aperturess of the hydrogel.

For further details of the hydrogel material for use in the present invention, and its preparation, please refer to the following publications: PCT Patent Applications Nos. WO-97/24149, WO-97/34947, WO-00/06214, WO-00/06215, WO-00/07638, WO-00/46319, WO-00/65143 and WO-01/96422.

### Hydrogel having embedded therein a reinforcement sheet of /iquid-water-permeable material

The layer of hydrogel prior to the introduction of apertures may suitably be present as a thin sheet (e.g. a sheet of 5 mm or less in thickness, e.g. 3 mm or less in thickness, e.g. 1 mm or less in thickness) having embedded therein a reinforcement sheet of liquid-water-permeable material, e.g. to provide mechanical strength. The reinforcement sheet of liquid-water-permeable material may, merely for brevity, be termed a reinforcement sheet herein. The reinforcement sheet may, for example, be or comprise a scrim, for example formed of a synthetic and/or natural polymer such as polyethylene or polypropylene or polyester or calcium alginate or carboxymethyl cellulose or any combination thereof. The scrim may be as described above.

According to the present invention, a polymeric film is disposed over, and optionally may contact, the non-wound-facing side of the layer of hydrogel, and the film has an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002. One or more further materials or layers, e.g. an absorbent layer as described herein, may be disposed between the polymeric film and the layer of hydrogel. The polymeric film may have an MVTR of from 1000 g/m²/24 hours to 3000 g/m²/24 hours, measured in accordance with BS EN 13726-2:2002. In an embodiment, the polymeric film is continuous in that it extends over and covers each of the apertures in the layer of hydrogel; such a polymeric film may be termed a first film herein.

In an embodiment, the polymeric film has apertures therein, at least some of which, optionally all of which, overlie the apertures in the layer of hydrogel and/or the polymeric film contacts the layer of hydrogel on the non-wound facing side thereof; such a film may be termed a second film herein. In an embodiment the apertures in the polymeric film are approximately the same size, shape and in the same arrangement as the apertures in the layer of hydrogel, such that each aperture in the hydrogel has a corresponding aperture in the polymeric layer.

In an embodiment, a further polymeric film, in addition to the first film, (which may also be termed a second film herein) is disposed over and in contact with the non-wound-facing side of the layer of hydrogel, the further polymeric film preferably having an MVTR of at least 100 g/m²/24 hours, optionally at least 200 g/m²/24 hours, optionally at least 300 g/m²/24 hours, optionally at least 500 g/m²/24 hours, optionally at least 600 g/m²/24 hours, measured in accordance with BS EN 13726-2:2002, and the polymeric films are adhered together by a layer of adhesive, and optionally the further polymeric film and/or the layer of adhesive has/have apertures therein, at least some of which, optionally all of which, overlie the apertures in the layer of hydrogel. In an embodiment, the layer of adhesive has apertures therein, at least some of which overlie the apertures the apertures in the layer of hydrogel and/or the apertures of the further polymeric film. In an embodiment the apertures in the layer of adhesive are approximately the same size, shape and in the same arrangement as the apertures in the layer of hydrogel and, if present, apertures in the polymeric film, such that each aperture in the layer of adhesive has a corresponding aperture in the hydrogel and/or the polymeric film.

In an embodiment, the composition or wound dressing comprises the layer of hydrogel, having in contact therewith a polymeric film having apertures therein (which may be termed a second film or apertured polymeric film herein), at least some of which, optionally all of which, overlie the apertures in the layer of hydrogel, an adhesive layer disposed on and in contact with the apertured polymeric film, and a continuous polymeric film (which may be termed a first film herein) is disposed on and in contact with the adhesive layer, the continuous polymeric film being continuous in that it extends over and covers each of the apertures in the layer of hydrogel and/or the apertures in the polymeric layer that is in contact with the layer of hydrogel.

The polymeric film(s), e.g. the first and/or second polymeric film, may (each) comprise or be a polyurethane film. Optionally, the polymeric film(s), e.g. the first and/or second polymeric film, which may (each) be or comprise a polyurethane film, is/are breathable. The polymeric film(s), e.g. the first and/or second polymeric film, may (each) have an MVTR of at least 10 g/m²/24 hours, optionally at least at least 50 g/m²/24 hours, optionally at least 100 g/m²/24 hours, optionally at least 200 gm²/24 hours, optionally at least 300 g/m²/24 hours, optionally at least 500 g/m²/24 hours, optionally at least 1000 g/m²/24 hours, with MVTR as measured by the method described in on BS EN 13726-2:2002. The polymeric film(s), e.g. the first and/or second polymeric film, may (each) have an MVTR of at least 10 g/m²/24 hours, optionally at least at least 50 g/m²/24 hours, optionally at least 100 g/m²/24 hours, optionally at least 200 gm²/24 hours, optionally at least 300 g/m²/24 hours, optionally at least 500 g/m²/24 hours, optionally at least 1000 g/m²/24 hours, with MVTR as measured by the method described in on BS EN 13726-2:2002. The polymeric fiim(s), e.g. the first and/or second polymeric film, may (each) have an MVTR of from 100 g/m²/24 hours to 10000 g/m²/24 hours, optionally an MVTR of from 100 g/m²/24 hours to 4000 g/m²/24 hours, optionally an MVTR of from 1000 g/m²/24 hours to 3000 g/m²/24 hours, optionally an MVTR of from 1500 to 2500 g/m²/24 hours.

In an embodiment, the composition or wound dressing further comprises a backing layer over the non-wound facing side of the apertured layer of hydrogel. The backing layer may be defined as the outermost layer of the composition or dressing on the non-wound-facing side of the composition. The first film described above may be or form part of the backing layer. The backing layer preferably provides a barrier to passage of microorganisms through the composition or dressing and further preferably blocks the escape of wound fluid from the dressing. The backing layer also provides for a reduction in the evaporation of moisture compared to when no backing layer is present. This reduction of the evaporation of moisture when combined with the water content of the hydrogel provides for the maintenance of a moist environment over the wound and overcomes the proposed problems of hydrogel layers with large diameter apertures.

The polymeric films(s), e.g. the first and/or second film, may (each) overlie at least some, optionally all, of the layer of hydrogel on the non-wound-facing side thereof.

The polymeric films(s), e.g. the first and/or second film, may (each), if desired, extend beyond the margins of the area occupied by the layer of hydrogel, and may be provided with a skin adhesive portion to secure the dressing to the skin. In an embodiment, the apertured hydrogel occupies an approximately central portion of the polymeric films(s), e.g. the first and/or second film, and the polymeric films(s), e.g. the first and/or second film, (each) extends beyond the central portion and the film(s) is/are provided with adhesive around the central portion, such that an adhesive seal can be formed when the dressing is placed on a skin of a user. The skin adhesive portion may be hydrogel in nature (for example a plasticised tacky hydrogel, which may be the same as or different from the hydrogel provided on the support member for the treatment according to the present invention), or may be another type of skin adhesive selected from the many skin adhesives known in the wound dressings art.

The precursor solution may be dispensed onto a substrate in a continuous or noncontinuous manner and the supporting layer structure placed on top, using the absorbancy characteristics of the support layer to take up at least in part the precursor solution.

The support structure in contact with the precursor solution may suitably be in the form of a layer. This layer may suitably be provided for the polymerisation on a surface, most preferably itself provided with a release layer such as siliconised paper of plastic. After polymerisation of such an arrangement, the resultant hydrogel/support layer composite will be in the form of a sheet having its underside protected by a release layer.

In another aspect, the composition of the present invention may be used as the wound contacting component of an electrode assembly for use in the electrical stimulation of wounds, with the wound-facing side of the layer of hydrogel facing, and optionally contacting, the wound during the electrical stimulation. The method involves passing a current through the hydrogel of the composition when the hydrogel is in electrical contact with the wound. The electrical stimulation may utilise alternating currents, direct currents or pulsed direct currents for example high voltage pulsed currents (HVPC). In a preferred embodiment the apertured hydrogel has an impedance measured at 500MHz of less than 50 Ohms, more preferably less than 20 Ohms and even more preferably less than 10 Ohms.

### Backing Layer

The backing layer may be defined as the outermost layer of the composition or dressing on the non-wound-facing side of the composition and may comprise a polymeric film as described herein, e.g. the first or second polymeric film as described above. The backing layer may be of a composite multilayered structure for example a film/foam laminate or film adhesive film laminate. The backing layer, e.g. the first and/or second polymeric film, preferably has a moisture transmission rate greater than 30 g/m²/24hr, more preferably greater than 300 g/m²/24hr and more preferably greater than 600g/m²/24hr and even more preferably greater than 1000g/m²/24h, an MVTR of from 1000 g/m²/24 hours to 3000 g/m²/24 hours. MVTR as mentioned elsewhere herein can be measured in accordance with BS EN 13726-2:2002. Preferably the polymeric film is a breathable and/or hydrophilic polyurethane.

An adhesive, for example an acrylic adhesive, may be coated onto the backing layer, e.g. the first and/or second film as described herein, and may be disposed on a wound-facing side of the backing layer. The adhesive may fully coat or only partially coat a side of, e.g. the wound-facing side of, the backing layer. When present only as a partial coating it is preferred that the adhesive forms a regular pattern. A partial coating may also be termed a discontinuous coating.

### Formation of Apertured Hydrogel

The apertures of hydrogel may be formed by first forming a sheet (or layer) of hydrogel and then cutting and removing a desired size and shape of holes from the sheet. Preferably the apertures are cut using a shaped die.

### Additional Absorbent Layer

An absorbent layer, which may be termed an additional absorbent layer since the hydrogel will act as an absorbent layer, maybe disposed on the wound-facing side of the layer of hydrogel. The absorbent layer may comprise a foam, preferably an open cell foam, preferably a hydrophilic aromatic or aliphatic polyurethane foam. The absorbent layer may comprise a fibrous material, preferably a fibrous material comprising fibres that swell in saline solution. Preferably the fibrous material is in the form of a non woven material and preferably the fibres of the fibrous material comprise an alginate, chitosan or a cellulose derivative or any combination thereof. The additional absorbent layer may also comprise a multilayer structure. The additional absorbent layer may also have a keying layer on the non wound facing side to attach it it to the wound facing side of the apertured layer of hydrogel. A keying layer may act to adhere two layers together, preferably by merging into one or both of the two adjacent layers it is adhering together.

An absorbent layer, which may be termed an additional absorbent layer since the hydrogel will act as an absorbent layer, may be disposed on the non-wound-facing side of the layer of hydrogel. The absorbent layer disposed on the non-wound-facing side of the layer of hydrogel may comprise a foam, preferably an open cell foam, preferably a hydrophile aromatic or aliphatic polyurethane foam. The absorbent layer disposed on the non-wound-facing side of the layer of hydrogel may comprise a fibrous material, preferably a fibrous material comprising fibres that swells in saline solution. Preferably the fibrous material is in the form of a non-woven material and preferably the fibres of the fibrous material comprise an alginate, chitosan or a cellulose derivative or any combination thereof. The additional absorbent layer disposed on the non-wound-facing side of the layer of hydrogel may also comprise a multilayer structure. The additional absorbent layer disposed on the non-wound-facing side of the layer of hydrogel may also have a keying layer on the wound facing side to attach it it to the non wound facing side of the apertured layer of hydrogel. A keying layer may act to adhere two layers together, preferably by merging into one or both of the two adjacent layers it is adhering together.

In an embodiment, an additional absorbent layer is disposed on each side of the layer of hydrogel, i.e. an additional absorbent layer is disposed on the wound-facing side of the layer of hydrogel and an additional absorbent layer is disposed on the non-wound-facing side of the layer of hydrogel. The additional absorbent layers may be the same as each other or different from one another and may each be as defined above.

When the backing layer, e.g. the first and/or second polymeric film as disclosed herein, is at least in part coated with adhesive and the backing layer extends beyond the perimeter of the apertured layer of hydrogel then the adhesive on the backing layer may adhere an additional absorbent layer (disposed on a wound-facing side of the hydrogel layer) to the backing layer and encapsulate the layer of hydrogel.

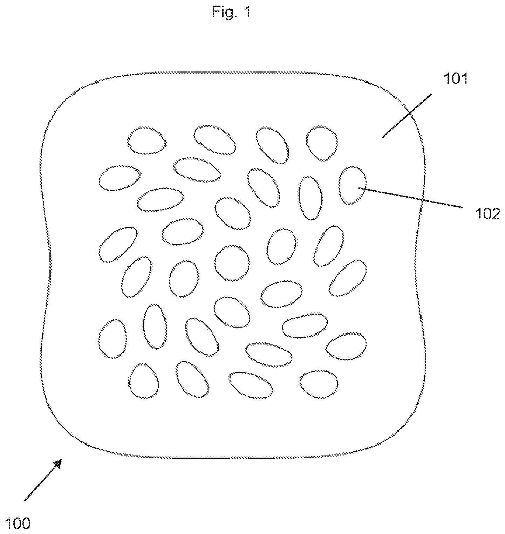

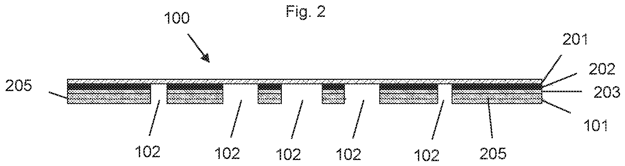

Figures 1 and 2 illustrate schematically embodiments of the dressing or composition (100) as described herein. Figure 1 shows an embodiment of the dressing or composition described herein, when viewed from an angle perpendicular to the plane of the layer of hydrogel (101), e.g. from above the wound-facing side of the layer of hydrogel. The apertures (102) are arranged in an array as shown. A backing layer (not shown in Figure 1) may be disposed on one side of the layer of hydrogel, e.g. the non-wound-facing side, the backing layer being continuous, in that it covers each of the apertures in the hydrogel, and extends over all of the hydrogel. The backing layer may, for example, be a breathable polymeric film, e.g. a breathable polyurethane film. A reinforcement sheet is embedded in the hydrogel (not shown in Figure 1, but illustrated in Figure 2).

Figure 2 shows schematically a cross-sectional view of an embodiment of the composition or wound dressing (100) described herein, In this embodiment, a layer of hydrogel (101) is shown, the hydrogel having apertures (102) extending therethrough. A reinforcement sheet (205) of liquid-water-permeable material in the form of a scrim is embedded within the layer of hydrogel (101) and has apertures therethrough corresponding to those in the surrounding hydrogel layer. A supporting film (203) overlies and is in contact with the hydrogel layer. An adhesive layer (202) overlies the supporting film. The supporting film (203) and the adhesive layer (202) each have apertures therein overlying the apertures (102) in the layer of hydrogel (101). The apertures in the supporting film (203) and adhesive (202) are of approximately the same size, shape and location as the apertures (102) in the layer of hydrogel. A backing film (401) is adhered to the adhesive layer (402), the backing layer being continuous, in that it covers each of the apertures in the hydrogel, and extends over all of the hydrogel.

The present invention will now be further described with reference to the following nonlimiting Examples.

### EXAMPLES

In these examples, the pre-gel formulations weres cured by a medium pressure mercury arc lamp (GEW, UK).

### Example 1 (reference)

Pre-gel: 67 parts by weight of 58% aqueous solution of the sodium salt of acrylamidomethylpropanesulphonic acid (Na AMPS, LZ2405 Lubrizol), 30 parts glycerol, 2 aperts acrylic acid and 0.1 parts of a 6 to 20 (by weight) mixture of Daracure 1173 photoinitiator (BASF) and IRR280 cross-linker (PEG400 diacrylate, UCB Chemicals).

### Example 2 (reference)

The pre-gel from example 1 was dispensed from a slot die onto a siliconised polyester film moving at 7m/minute to a coat weight of circa 0.6 kg/m². A 20 gsm non woven polypropylene scrim (RKW) was then placed on top of the pre-gel and then passed under a medium pressure mercury arc lamp (GEW, UK) to cure and then a further circa 0.6 kg/m² pre-gel from example 1 was dispensed from a second slot die onto the 20 gsm non woven polypropylene scrim (RKW) and then passed under a second medium pressure mercury arc lamp (GEW, UK) to cure and to form a sheet (or layer) of hydrogel with an embedded reinforced layer hydrogel sheet.

### Example 3 (reference)

A 100mmX100mm sheet of reinforced hydrogel from example 2 was laminated to a 30µm polyurethane film (Inspire 2352, Coveris UK).

### Example 4

7mm diameter circles, 4mm separation from the edge of one circle to the next, were cut from the sheet (100mmx100mm) produced in Example 2 in a centrally positioned array comprising 35 apertures The perimeter of the array was 70mm long by 70mm wide. The apertured hydrogel sheet was then laminated to a square piece of a 30µm polyurethane film. The mean area of the apertures was 38.48 mm².

### Example 5 (reference)

7mm diameter circles, 4mm separation from the edge of one circle to the next, were cut from a 100mmX100mm sheet of fully adhesive coated polyurethane film (RAP10464A, Rayleigh Coatings, UK) in a centrally positioned array comprising 35 apertures The perimeter of the array was 70mm long by 70mm wide. A 100mmx100mm sheet of hydrogel from Example 2 was laminated to the polyurethane side of the adhesive coated polyurethane film. A 100mmx100mm sheet of a non adhesive coated polyurethane film (Inspire 2352, Coveris UK) was then laminated onto the adhesive side of the adhesive coated polyurethane film. The mean area of the apertures in the adhesive coated film was 38.48 mm².

### Example 6

A 100mmx100mm sheet of hydrogel from Example 2 was laminated to the polyurethane side of a 100mmX100mm sheet of fully adhesive coated polyurethane film (RAP10464A, Rayleigh Coatings, UK).

7mm diameter circles, 4mm separation from the edge of one circle to the next, were cut from the sheet produced above (100mmx100mm) in a centrally positioned array comprising 35 apertures The perimeter of the array was 70mm long by 70mm wide. A 100mmx100mm sheet of a non adhesive coated polyurethane film (Inspire 2352, Coveris UK) was then laminated onto the adhesive side of the adhesive coated polyurethane film. The mean area of the apertures was 38.48 mm²

### Example 7

Fluid Handling Capacity for the samples taken from the centrally placed apertured region of the sheet hydrogels produced in Examples 3, 4, 5, 6. Measurements were undertaken according to *BS EN 13726-1:2002: Test methods for primary wound dressings. Aspects of absorbency. Section 3.3 Fluid handling capacity.*

| Sample | Average Sample Weight | Average Fluid Handling Capacity |
|---|---|---|
| | g | g/m2(24hr)(standard deviation) |
| Example 3 (Non apertured) | 1.09 | 16643 (497) |

| | | |
|---|---|---|
| Example 4 (Apertured) | 0.78 | 19453 (288) |
| Example 5 (Non apertured) | 1.23 | 9435 (106) |
| Example 6 (Apertured) | 0.85 | 9895 (417) |

The average fluid handling capacities of the apertured and non apertured hydrogel sheets are at least comparable. The greater average fluid handling capacity in examples 3 and 4 arises from the presence of only a high MVTR film. In examples 5 and 6 there is in addition to the high MVTR backing film an acrylic adhesive coated polyurethane film acting as a support layer.

## Claims

1. A composition comprising:
a layer of water-swellable hydrogel, wherein the hydrogel has embedded therein a reinforcement sheet of liquid-water-permeable material,
wherein the layer of hydrogel has a wound-facing side and a non-wound-facing side and apertures extend through the layer of hydrogel from the wound-facing side to the non-wound-facing side and through the reinforcement sheet,
wherein the mean area of the apertures on at least one of the wound-facing side and non-wound-facing side of the hydrogel is at least 10 mm²,
wherein a polymeric film is disposed over the non-wound-facing side of the layer of hydrogel, the film having an MVTR of at least 100 g/m²/24 hours, measured in accordance with BS EN 13726-2:2002.

2. The composition according to claim 1, wherein the total area occupied by the apertures on the wound-facing side and/or non-wound-facing side of the layer of hydrogel is 60% or less of the total area of the layer of hydrogel.

3. The composition according to claim 1 or 2, wherein the layer of hydrogel has from about 1 to about 5 apertures per square 10 cm of the wound-facing side and/or non-wound-facing side.

4. The composition according to any one of the preceding claims, wherein the mean area of the apertures on at least one of the wound-facing side and non-wound-facing side of the layer of hydrogel is at least 10 mm²,
the layer of hydrogel has from about 1 to about 5 apertures per square 10 cm of the wound-facing side and/or non-wound-facing side,
the hydrogel is an ionic hydrogel, and
a polymeric film is disposed over the non-wound-facing side of the layer of hydrogel, the film having an MVTR of at least 100 g/m²/24 hours, measured in accordance with BS EN 13726-2:2002.

5. The composition according to any one of the preceding claims, wherein the polymeric film has an MVTR of from 1000 g/m²/24 hours to 3000 g/m²/24 hours, measured in accordance with BS EN 13726-2:2002.

6. The composition according to any one of the preceding claims, wherein the polymeric film is continuous in that it extends over and covers each of the apertures in the layer of hydrogel.

7. The composition according to any one of the preceding claims, wherein an absorbent layer is disposed between the layer of hydrogel and the polymeric film and, wherein the absorbent layer may comprise a foamed hydrophilic material, and wherein the foamed hydrophilic material may be selected from a foamed polyurethane and a foamed hydrogel.

8. The composition according to any one of claims 1 to 5, wherein a further polymeric film is disposed over and in contact with the non-wound-facing side of the layer of hydrogel, the further polymeric film having an MVTR of at least 100 g/m²/24 hours, measured in accordance with BS EN 13726-2:2002, and the polymeric films are adhered together by a layer of adhesive.

9. The composition according to claim 8, wherein the further polymeric film has apertures therein, at least some of which overlie the apertures in the layer of hydrogel, and the polymeric film disposed further away from the non-wound-facing side of the layer of hydrogel is continuous in that it extends over and covers each of the apertures in the layer of hydrogel and/or the further polymeric layer, and optionally, wherein the layer of adhesive has apertures therein, at least some of which overlie the apertures in the layer of hydrogel and/or the apertures of the further polymeric film.

10. The composition according to any one of the preceding claims, wherein the layer of hydrogel is 3 mm or less in thickness, measured from the wound-facing side to the non-wound-facing side.

11. A method of forming a composition comprising:
providing a layer of water-swellable hydrogel, wherein the hydrogel has embedded therein a reinforcement sheet of liquid water-permeable material, the layer of hydrogel having a wound-facing side and a non-wound-facing side;
cutting apertures through the layer of water-swellable hydrogel and the reinforcement sheet embedded therein to form apertures that extend through the layer of hydrogel from the wound-facing side to the non-wound-facing side and through the reinforcement sheet,
wherein the mean area of the apertures on at least one of the wound-facing side and non-wound-facing side of the hydrogel is at least 10 mm²,
wherein the method involves placing a polymeric film over the non-wound-facing side of the layer of hydrogel, the film having an MVTR of at least 100 g/m²/24 hours, measured in accordance with BS EN 13726-2:2002.

12. The method according to claim 11, wherein the method involves die cutting the apertures and/or wherein the polymeric film is continuous in that it extends over and covers each of the apertures in the layer of hydrogel.

13. The method according to claim 11, wherein either the polymeric film is placed over the non-wound-facing side of the layer of hydrogel before the cutting of the apertures in the layer of hydrogel and apertures are cut in both the polymeric film and the layer of hydrogel or the polymeric film is placed over the non-wound-facing side of the layer of hydrogel after the cutting of the apertures in the layer of hydrogel and the polymeric film has apertures therethrough, at least some of which overlie the apertures of the layer of hydrogel, and optionally wherein the method further involves adhering a continuous polymeric film to the apertured polymeric film, the continuous polymeric film having an MVTR of at least 100 g/m²/24 hours, measured in accordance with BS EN 13726-2:2002, wherein the polymeric film is continuous in that it extends over and covers each of the apertures in the layer of hydrogel and/or the apertured polymeric film.

14. The method according to claim 13, wherein the continuous polymeric film is adhered to the apertured polymeric film by means of a layer of adhesive, the layer of adhesive has apertures therein, at least some of which overlie the apertures in the layer of hydrogel and/or the apertures of the apertured polymeric film, and optionally wherein the continuous polymeric film is adhered to the apertured polymeric film by means of a layer of adhesive, the layer of adhesive being present on the polymeric film that is placed over the non-wound-facing side of the layer of hydrogel at the time at which holes are cut in the polymeric film, the holes being cut in both the polymeric film and the adhesive.

15. The method according to claim 11 or claim 12, wherein the method further involves placing an absorbent layer over the non-wound-facing side of the layer of hydrogel, , the absorbent layer being disposed closer to the non-wound-facing side of the layer of hydrogel than the polymeric film.

## Patentansprüche

1. Zusammensetzung, umfassend:
eine Schicht aus wasserquellbarem Hydrogel, wobei das Hydrogel eine darin eingebettete Verstärkungslage aus Material aufweist, das für flüssiges Wasser durchlässig ist,
wobei die Hydrogelschicht eine der Wunde zugewandte Seite und eine nicht der Wunde zugewandte Seite aufweist und sich Öffnungen durch die Hydrogelschicht von der der Wunde zugewandten Seite zu der nicht der Wunde zugewandten Seite und durch die Verstärkungslage erstrecken,
wobei die mittlere Fläche der Öffnungen auf mindestens einer von der der Wunde zugewandten Seite und der nicht der Wunde zugewandten Seite des Hydrogels mindestens 10 mm² ist,
wobei ein Polymerfilm über der nicht der Wunde zugewandten Seite der Hydrogelschicht angeordnet ist, wobei der Film einen MVTR von mindestens 100 g/m²/24 Stunden aufweist, gemessen gemäß BS EN 13726-2:2002.

2. Zusammensetzung gemäß Anspruch 1, wobei die Gesamtfläche, die von den Öffnungen auf der der Wunde zugewandten Seite und/oder der nicht der Wunde zugewandten Seite der Hydrogelschicht eingenommen wird, 60 % oder weniger der Gesamtfläche der Hydrogelschicht ist.

3. Zusammensetzung gemäß Anspruch 1 oder 2, wobei die Hydrogelschicht der der Wunde zugewandten Seite und/oder der nicht der Wunde zugewandten Seite von etwa 1 bis etwa 5 Öffnungen pro 10 cm² aufweist.

4. Zusammensetzung gemäß einem der vorherigen Ansprüche, wobei die mittlere Fläche der Öffnungen auf mindestens einer von der der Wunde zugewandten Seite und der nicht der Wunde zugewandten Seite der Hydrogelschicht mindestens 10 mm² ist,
die Hydrogelschicht pro 10 cm² der der Wunde zugewandten Seite und der nicht der Wunde zugewandten Seite von etwa 1 bis etwa 5 Öffnungen aufweist,
das Hydrogel ein ionisches Hydrogel ist, und
ein Polymerfilm über der nicht der Wunde zugewandten Seite der Hydrogelschicht aufgebracht ist,
wobei der Film einen MVTR von mindestens 100 g/m²/24 Stunden aufweist, gemessen gemäß BS EN 13726-2:2002.

5. Zusammensetzung gemäß einem der vorherigen Ansprüche, wobei der Polymerfilm einen MVTR von 1000 g/m²/24 Stunden bis 3000 g/m²/24 Stunden aufweist, gemessen gemäß BS EN 13726-2:2002.

6. Zusammensetzung gemäß einem der vorherigen Ansprüche, wobei der Polymerfilm dahingehend kontinuierlich ist, dass er sich über jede der Öffnungen in der Hydrogelschicht erstreckt und diese abdeckt.

7. Zusammensetzung gemäß einem der vorherigen Ansprüche, wobei eine absorbierende Schicht zwischen der Hydrogelschicht und dem Polymerfilm angeordnet ist, und wobei die absorbierende Schicht ein geschäumtes hydrophiles Material umfassen kann, und wobei das geschäumte hydrophile Material aus einem geschäumten Polyurethan und einem geschäumten Hydrogel ausgewählt sein kann.

8. Zusammensetzung gemäß einem der Ansprüche 1 bis 5, wobei ein weiterer Polymerfilm über und in Kontakt mit der nicht der Wunde zugewandten Seite der Hydrogelschicht angeordnet ist, wobei der weitere Polymerfilm einen MVTR von mindestens 100 g/m²/24 Stunden aufweist, gemessen gemäß BS EN 13726-2:2002, und die Polymerfilme durch eine Klebstoffschicht aneinandergeklebt sind.

9. Zusammensetzung gemäß Anspruch 8, wobei der weitere Polymerfilm Öffnungen darin aufweist, wovon mindestens einige über den Öffnungen in der Hydrogelschicht liegen, und der Polymerfilm, der weiter weg von der nicht der Wunde zugewandten Seite der Hydrogelschicht angeordnet ist, dadurch kontinuierlich ist, dass er sich über jede der Öffnungen in der Hydrogelschicht und/oder der weiteren Polymerschicht erstreckt und diese abdeckt, und optional, wobei die Klebstoffschicht Öffnungen darin aufweist, wovon mindestens einige über den Öffnungen in der Hydrogelschicht und/oder den Öffnungen des weiteren Polymerfilms liegen.

10. Zusammensetzung gemäß einem der vorherigen Ansprüche, wobei die Hydrogelschicht 3 mm oder weniger stark ist, gemessen von der der Wunde zugewandten Seite zu der nicht der Wunde zugewandten Seite.

11. Verfahren zum Bilden einer Zusammensetzung, umfassend:
Bereitstellen einer Schicht aus wasserquellbarem Hydrogel, wobei das Hydrogel eine darin eingebettete Verstärkungslage aus Flüssigkeit-Wasser-durchlässigem Material aufweist, wobei die Hydrogelschicht eine der Wunde zugewandte Seite und eine nicht der Wunde zugewandte Seite aufweist;
Schneiden von Öffnungen durch die Schicht aus wasserquellbarem Hydrogel und die darin eingebettete Verstärkungslage, um Öffnungen zu bilden, die sich von der der Wunde zugewandten Seite zu der nicht der Wunde zugewandten Seite durch die Hydrogelschicht und durch die Verstärkungslage erstrecken,
wobei die mittlere Fläche der Öffnungen auf mindestens einer von der der Wunde zugewandten Seite und der nicht der Wunde zugewandten Seite des Hydrogels mindestens 10 mm² ist,
wobei das Verfahren ein Platzieren eines Polymerfilms über der nicht der Wunde zugewandten Seite der Hydrogelschicht involviert, wobei der Film einen MVTR von mindestens 100 g/m²/24 Stunden aufweist, gemessen gemäß BS EN 13726-2:2002.

12. Verfahren gemäß Anspruch 11, wobei das Verfahren ein Stanzen der Öffnungen beinhaltet und/oder wobei der Polymerfilm dahingehend kontinuierlich ist, dass er sich über jede der Öffnungen in der Hydrogelschicht erstreckt und diese abdeckt.

13. Verfahren gemäß Anspruch 11, wobei entweder der Polymerfilm vor dem Schneiden der Öffnungen in der Hydrogelschicht über die nicht zum Wickeln gerichtete Seite der Hydrogelschicht platziert wird und Öffnungen sowohl in den Polymerfilm als auch in die Hydrogelschicht geschnitten werden, oder der Polymerfilm nach dem Schneiden der Öffnungen in der Hydrogelschicht über die nicht zum Wickeln gerichtete Seite der Hydrogelschicht gelegt wird und der Polymerfilm Öffnungen dort hindurch aufweist, wovon mindestens einige über den Öffnungen der Hydrogelschicht liegen, und optional wobei das Verfahren ferner ein Kleben eines kontinuierlichen Polymerfilms an den geöffneten Polymerfilm involviert, wobei der kontinuierliche Polymerfilm einen MVTR von mindestens 100 g/m²/24 Stunden aufweist, gemessen gemäß BS EN 13726-2:2002, wobei der Polymerfilm dahingehend kontinuierlich ist, dass er sich über jede der Öffnungen in der Hydrogelschicht und/oder dem mit Öffnungen versehenen Polymerfilm erstreckt und diese abdeckt.

14. Verfahren gemäß Anspruch 13, wobei der kontinuierliche Polymerfilm mittels einer Klebstoffschicht an den geöffneten Polymerfilm geklebt ist, wobei die Klebstoffschicht Öffnungen darin aufweist, wovon mindestens einige über den Öffnungen in der Hydrogelschicht und/oder den Öffnungen des geöffneten Polymerfilms liegen, und optional wobei der kontinuierliche Polymerfilm mittels einer Klebstoffschicht an den geöffneten Polymerfilm geklebt wird, wobei die Klebstoffschicht an dem Polymerfilm vorhanden ist, der zu dem Zeitpunkt, an dem Löcher in den Polymerfilm geschnitten werden, über die nicht zum Wickeln gerichtete Seite der Hydrogelschicht gelegt wird, wobei die Löcher sowohl in den Polymerfilm als auch in den Klebstoff geschnitten werden.

15. Verfahren gemäß Anspruch 11 oder 12, wobei das Verfahren ferner ein Platzieren einer absorbierenden Schicht über der nicht der Wunde zugewandten Seite der Hydrogelschicht involviert, wobei die absorbierende Schicht näher an der nicht der Wunde zugewandten Seite der Hydrogelschicht angeordnet ist als der Polymerfilm.

## Revendications

1. Une composition comprenant :
une couche d'hydrogel gonflable à l'eau, dans laquelle l'hydrogel a intégré dans celle-ci une feuille de renforcement de matériau perméable à l'eau liquide,
dans laquelle la couche d'hydrogel a un côté faisant face à la blessure et un côté ne faisant pas face à la blessure et des ouvertures s'étendent à travers la couche d'hydrogel à partir du côté faisant face à la blessure vers le côté ne faisant pas face à la blessure et à travers la feuille de renforcement,
dans laquelle la zone moyenne des ouvertures sur au moins un du côté faisant face à la blessure et du côté ne faisant pas face à la blessure de l'hydrogel est d'au moins 10 mm²,
dans laquelle un film polymère est disposé sur le côté ne faisant pas face à la blessure de la couche d'hydrogel, le film ayant un MVTR (taux de transmission de vapeur d'eau) d'au moins 100 g/m²/24 heures, mesuré selon la norme BS EN 13726-2:2002.

2. La composition selon la revendication 1, dans laquelle la zone totale occupée par les ouvertures sur le côté faisant face à la blessure et / ou le côté ne faisant pas face à la blessure de la couche d'hydrogel est de 60 % ou moins de la zone totale de la couche d'hydrogel.

3. La composition selon la revendication 1 ou 2, dans laquelle la couche d'hydrogel a d'environ 1 à environ 5 ouvertures par 10 cm carrés du côté faisant face à la blessure et / ou du côté ne faisant pas face à la blessure.

4. La composition selon l'une quelconque des revendications précédentes, dans laquelle la zone moyenne des ouvertures sur au moins un du côté faisant face à la blessure et du côté ne faisant pas face à la blessure de la couche d'hydrogel est d'au moins 10 mm²,
la couche d'hydrogel a d'environ 1 à environ 5 ouvertures par 10 cm carrés du côté faisant face à la blessure et / ou du côté ne faisant pas face à la blessure, l'hydrogel est un hydrogel ionique, et
un film polymère est disposé sur le côté ne faisant pas face à la blessure de la couche d'hydrogel, le film ayant un MVTR d'au moins 100 g/m²/24 heures, mesuré selon la norme BS EN 13726-2:2002.

5. La composition selon l'une quelconque des revendications précédentes, dans laquelle le film polymère a un MVTR de 1000 g/m²/24 heures à 3000 g/m²/24 heures, mesuré selon la norme BS EN 13726-2:2002.

6. La composition selon l'une quelconque des revendications précédentes, dans laquelle le film polymère est continu en ce qu'il s'étend sur et couvre chacune des ouvertures dans la couche d'hydrogel.

7. La composition selon l'une quelconque des revendications précédentes, dans laquelle une couche absorbante est disposée entre la couche d'hydrogel et le film polymère et, dans laquelle la couche absorbante peut comprendre un matériau hydrophile en mousse, et dans laquelle le matériau hydrophile en mousse peut être choisi parmi un polyuréthane en mousse et un hydrogel en mousse.

8. La composition selon l'une quelconque des revendications 1 à 5, dans laquelle un film polymère supplémentaire est disposé sur et en contact avec le côté ne faisant pas face à la blessure de la couche d'hydrogel, le film polymère supplémentaire ayant un MVTR d'au moins 100 g/m²/24 heures, mesuré selon la norme BS EN 13726-2:2002, et les films polymères sont collés ensemble par une couche d'adhésif.

9. La composition selon la revendication 8, dans laquelle le film polymère supplémentaire a des ouvertures dans celui-ci, dont au moins certaines recouvrent les ouvertures dans la couche d'hydrogel, et le film polymère disposé plus loin du côté ne faisant pas face à la blessure de la couche d'hydrogel est continu en ce qu'il s'étend sur et couvre chacune des ouvertures dans la couche d'hydrogel et /ou la couche polymère supplémentaire, et éventuellement, dans laquelle la couche d'adhésif a des ouvertures dans celle-ci, dont au moins certaines recouvrent les ouvertures dans la couche d'hydrogel et / ou les ouvertures du film polymère supplémentaire.

10. La composition selon l'une quelconque des revendications précédentes, dans laquelle la couche d'hydrogel a une épaisseur de 3 mm ou moins, mesurée à partir du côté faisant face à la blessure vers le côté ne faisant pas face à la blessure.

11. Un procédé de formation d'une composition comprenant :
la fourniture d'une couche d'hydrogel gonflable à l'eau, dans lequel l'hydrogel a intégré dans celle-ci une feuille de renforcement de matériau liquide perméable à l'eau, la couche d'hydrogel ayant un côté faisant face à la blessure et un côté ne faisant pas face à la blessure ;
la découpe des ouvertures à travers la couche d'hydrogel gonflable à l'eau et la feuille de renforcement intégrée dans celle-ci pour former des ouvertures qui s'étendent à travers la couche d'hydrogel à partir du côté faisant face à la blessure vers le côté ne faisant pas face à la blessure et à travers la feuille de renforcement,
dans lequel la zone moyenne des ouvertures sur au moins un du côté faisant face à la blessure et du côté ne faisant pas face à la blessure de l'hydrogel est d'au moins 10 mm²,
dans lequel le procédé consiste à placer un film polymère sur le côté ne faisant pas face à la blessure de la couche d'hydrogel, le film ayant un MVTR d'au moins 100 g/m²/24 heures, mesuré selon la norme BS EN 13726-2:2002.

12. Le procédé selon la revendication 11, dans lequel le procédé consiste à découper à l'emporte-pièce les ouvertures et / ou dans lequel le film polymère est continu en ce qu'il s'étend sur et couvre chacune des ouvertures dans la couche d'hydrogel.

13. Le procédé selon la revendication 11, dans lequel soit le film polymère est placé sur le côté ne faisant pas face à la blessure de la couche d'hydrogel avant la découpe des ouvertures dans la couche d'hydrogel et les ouvertures sont découpées à la fois dans le film polymère et la couche d'hydrogel ou le film polymère est placé sur le côté ne faisant pas face à la blessure de la couche d'hydrogel après la découpe des ouvertures dans la couche d'hydrogel et le film polymère a des ouvertures à travers celui-ci, dont au moins certaines recouvrent les ouvertures de la couche d'hydrogel, et éventuellement dans lequel le procédé consiste en outre à coller un film polymère continu au film polymère perforé, le film polymère continu ayant un MVTR d'au moins 100 g/m²/24 heures, mesuré selon la norme BS EN 13726-2:2002, dans lequel le film polymère est continu en ce qu'il s'étend sur et couvre chacune des ouvertures dans la couche d'hydrogel et / ou le film polymère perforé.

14. Le procédé selon la revendication 13, dans lequel le film polymère continu est collé au film polymère perforé au moyen d'une couche d'adhésif, la couche d'adhésif a des ouvertures dans celle-ci, dont au moins certaines recouvrent les ouvertures dans la couche d'hydrogel et / ou les ouvertures du film polymère perforé, et éventuellement dans lequel le film polymère continu est collé au film polymère perforé au moyen d'une couche d'adhésif, la couche d'adhésif étant présente sur le film polymère qui est placé sur le côté ne faisant pas face à la blessure de la couche d'hydrogel au moment où des trous sont découpés dans le film polymère, les trous étant découpés à la fois dans le film polymère et dans l'adhésif.

15. Le procédé selon la revendication 11 ou la revendication 12, dans lequel le procédé consiste en outre à placer une couche absorbante sur le côté ne faisant pas face à la blessure de la couche d'hydrogel, la couche absorbante étant disposée plus près du côté ne faisant pas face à la blessure de la couche d'hydrogel que le film polymère.
